# EUROPEAN PATENT SPECIFICATION

(11) **EP 2 386 855 B1**
(45) Date of publication and mention of the grant of the patent: **16.10.2013**
(21) Application number: 11003641.5
(22) Date of filing: 04.05.2011
(51) Int. Cl.: G01N 33/487, A61B 5/145

(54) **Hermetically sealed test strip container**
Hermetisch versiegelter Teststreifenbehälter
Récipient de bandelette réactive fermé hermétiquement

(30) Priority: 11.05.2010 US 777339
(43) Date of publication of application: 16.11.2011
(73) Proprietor: Roche Diagnostics GmbH, 68305 Mannheim (DE); F.Hoffmann-La Roche AG, 4070 Basel (CH)
(72) Inventor: Chan, Frank A., Sunnyvale, CA 94087 (US); Wiegel, Chris, Sunnyvale, CA 94086 (US)

(56) References cited:
- US-A1- 2004 178 216
- US-A1- 2006 182 656
- US-A1- 2007 293 790
- US-A1- 2008 131 322
- US-B1- 6 508 380

## Description

### Technical Field

Embodiments of the present disclosure generally relate to a test strip container.

### Background

As the number of patients suffering from diabetes and similar medical conditions increases, self-monitoring of blood glucose, wherein the patient monitors his or her blood glucose levels independently has become common practice. A person having type 1 diabetes could perform an average of 5 to 10 bG tests per day, thus, dedicating a great amount of time from their day to testing. Therefore, the total test time to perform a single test strip from start to finish becomes very important.

In US 2006/182656 a dispenser for flattened articles is shown, wherein a plunger which can be moved by a spring is used to expel these flattened articles from the dispenser through an opening.

US 2007/293790 discloses a system for determining a substance, wherein test strips are provided. The test strips are stored in a cavity, and can be expelled by a plunger, the plunger is biased by a spring.

### Summary

In accordance with one embodiment of the present disclosure, a test strip container comprising a housing defining a cavity having a dispensing wall, a housing inlet, and a dispensing outlet is presented. The container also comprises a lid mounted movably on the housing to define a primed position and an unprimed position therebetween. A cartridge provided within the cavity holds a plurality of test strips. The cartridge has a plunger entrance seal and a strip exit seal. A biasing member disposed within the cartridge urges the plurality of test strips towards the dispensing wall to be aligned with the dispensing oudet. A plunger is oriented to open the plunger entrance seal through the housing inlet when the lid is in the primed position and to urge a single test strip from the plurality of test strips through the strip exit seal of the cartridge and the dispensing outlet of the housing when the lid is transitioned from the primed position to the unprimed position.

In accordance with another embodiment of the present disclosure, a test strip container comprises a housing defining a cavity, a lid mounted movably on the housing to define a primed position and an unprimed position therebetween, a slidable seal positioned on the housing and providing a hermetic seal to a volume defined by the cavity of the housing and the lid, a cartridge provided within the cavity and holding a plurality of test strips. The cartridge has a dispensing wall and a dispensing outlet. A biasing member is positioned in the cartridge urging the plurality of test strips towards the dispensing wall, and a plunger is oriented to enter the cartridge and urge a single test strip from the plurality of test strips through the dispensing outlet when the lid is transitioned from the primed position to the unprimed position.

In accordance with another embodiment, a test strip container comprises a housing defining a cavity with a housing inlet, a dispensing outlet, and a sliding member mounted movably on the housing and providing a primed position and an unprimed position. A cartridge is provided within the cavity and has a dispensing wall, the cartridge holds a plurality of test strips. A biasing member is disposed within the cartridge and urges the plurality of test strips toward the dispensing outlet. A plunger is operatively connected to the sliding member and positioned to enter the cartridge through the housing inlet when the sliding member is in the primed position and to urge a single test strip from the plurality of test strips through the dispensing outlet when the sliding member is transitioned from the primed position to the unprimed position.

In accordance with another embodiment, not forming part of the invention of the present disclosure, a test strip container comprises a housing accommodating a plurality of test strips therein. The housing has a dispensing wall with a dispensing outlet. The container comprises a biasing member provided within the housing and urges the plurality of test strips towards the dispensing wall. A lever actuator is provided in the housing and has a primed position and an unprimed position. The lever has an actuation side and a lifting side. The lever actuator is connected to the lever on the actuation side. The lever on the lifting side urges a single test strip from the plurality of test strips through the dispensing outlet when the lever actuator is transitioned from the primed position to the unprimed position.

### Brief Description of the several views of the drawings

The following detailed description of specific embodiments of the present disclosure can be best understood when read in conjunction with the following drawings, where like structure is indicated with like reference numerals and in which:
Fig. 1A shows an upper side perspective view of a test strip container in accordance with one embodiment, not forming part of the invention, defining a primed position;
Fig. 1B shows an upper side perspective view of a test strip container in accordance with one embodiment not forming part of the invention defining an unprimed position;
Fig. 2 shows a cross-sectional view of a test strip container in accordance with Fig. 1A;
Fig. 3 shows a cross-sectional view of a test strip container in accordance with an embodiment of Fig. 1B, not forming part of the invention;
Fig. 4 shows a cross-sectional view of a test strip container in accordance with another embodiment;
Fig. 5 shows a cross-sectional view of a test strip container in accordance with yet another embodiment;
Fig. 6A shows a side view of a test strip container in accordance with yet another embodiment, not forming part of the invention;
Fig. 6B shows a top view of the test strip container of Fig. 6A;
Fig. 7A shows a schematic view of a test strip container in accordance with yet another embodiment, not forming part of the invention;
Fig. 7B shows a schematic view of a test strip container in accordance with the embodiment of Fig. 7A, not forming part of the invention; and
Fig. 8 shows a perspective view of a test strip container in accordance with another embodiment not forming part of the invention.

### Detailed Description

According to the present disclosure a test strip container (5) for a plurality of test strips (24) is provided comprising:
a housing (10) defining a cavity (12) which holds the plurality of test strips (24);
a dispensing outlet (20);
a dispensing wall (34);
a priming means (14, 60, 82) mounted movably on the housing (10) to define a primed position and an unprimed position therebetween;
a biasing member (22) which urges the plurality of test strips (24) towards the dispensing wall (34) to be aligned with the dispensing outlet (20); and
urging means (30, 80) oriented to urge a single test strip (24) from the plurality of test strips (24) through the dispensing outlet (20) when the priming means (14, 60, 82) is transitioned from the primed position to the unprimed position.

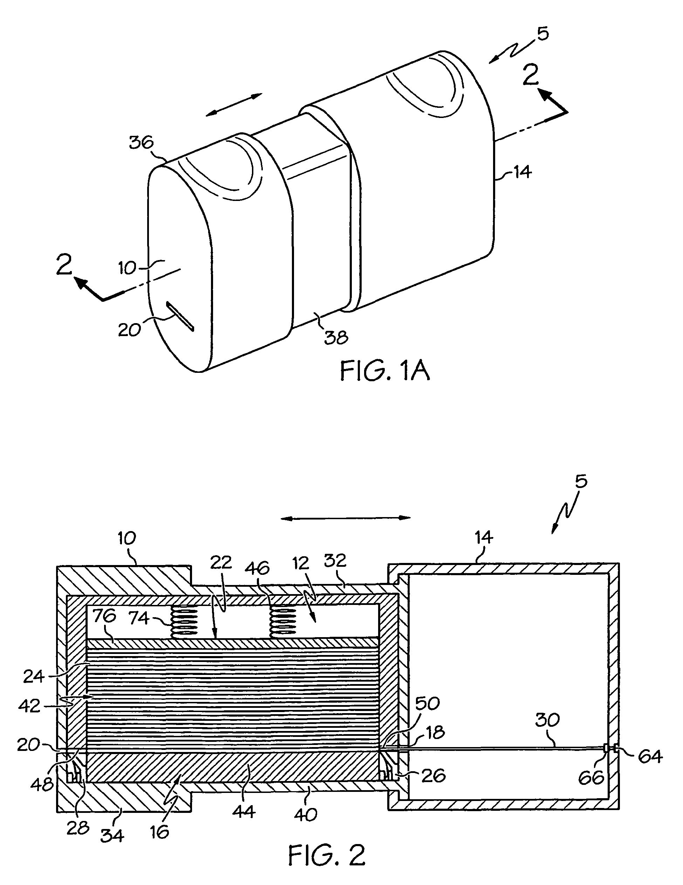

Referring to Fig. 1A, in accordance with one embodiment, not forming part of the invention, a test strip container 5 is provided for storing a plurality of test strips. Referring to Fig. 2, the test strip container 5 comprises a housing 10 that defines a cavity 12. The container 5 also comprises a lid 14 mounted on the housing 10 to provide for relative motion to define a primed position and an unprimed position. The housing 10 comprises a cartridge 16, a housing inlet 18, a dispensing outlet 20, and a biasing member 22. The cartridge 16 is provided within the cavity 12, where the cartridge 16 holds a plurality of test strips 24. The cartridge 16 comprises a plunger entrance seal 26 and a strip exit seal 28.

The container 5 also comprises a plunger 30 disposed through the housing inlet 18. The plunger 30 is oriented to open the plunger entrance seal 26, and urge a single test strip 24 through the strip exit seal 28 and the dispensing outlet 20 when the container 5 is in the primed position (Fig. 1A), The cavity 12 may have a cavity biasing wall 32 and a cavity dispensing wall 34. The biasing member 22 may be disposed within the cartridge 16, and may urge the plurality of test strips 24 towards the cavity dispensing wall 34.

The container 5 disclosed herein provides a substantially moisture-proof, air-tight apparatus for dispensing diagnostic test strips. The container holds a plurality of test strips and dispenses one strip at a time. Thus, the apparatus is easily utilized for dispensing a single diagnostic test strip and provides a convenient means for storing test strips and testing fluids using the strip.

In further describing the embodiments of the present disclosure, a conventional test strip 24 may be referenced. Examples of such test strips suitable for use with the subject disclosure include those described in U.S. Patent Nos. 6,193,873; 6,475,372; 6,716,577; 6,620,310; and 6,558,528. The length of the test strip generally ranges from about 3 mm to about 1000 mm, or from about 10 mm to about 100 mm, or from about 20 mm to about 60 mm. Although the container is discussed in reference to dispensing test strips, it is also contemplated that the container may be used to dispense other items having a substantially rectangular shape.

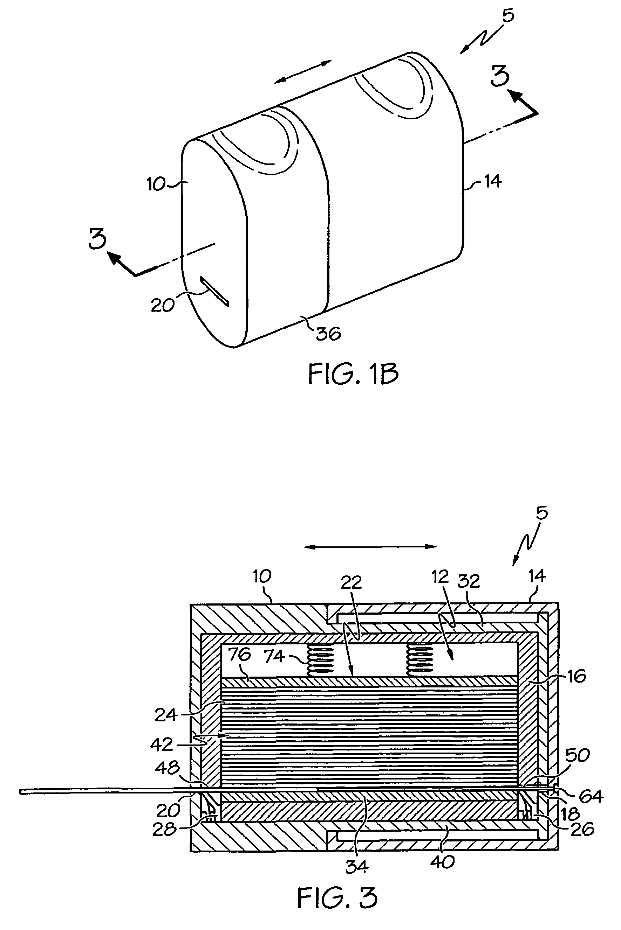

Referring to Figs. 1A and 1B, the test strip container 5 may comprise a housing 10. In one embodiment, not forming part of the invention, the housing 10 may have a substantially rectangular shape. The housing 10 may be sized and contoured to fit comfortably in a user's hands, and provide for easy dispensing of a single test strip 24 from the container 5. The housing 10 may also comprise other shapes and sizes.

Referring to Fig. 2, the internal volume of the housing 10 may define a cavity 12. The cavity 12 extends from the base of the housing 10 to the top opening (not shown) of the housing 10. The cavity may have two longitudinal walls, provided opposite one another, the cavity biasing wall 32 and the cavity dispensing wall 34. The cavity dispensing wall 34 and the cavity biasing wall 32 may be disposed in a parallel relation in one embodiment, not forming part of the invention.

Referring to Figs. 1A and 1B, the housing may comprise a housing shoulder 36 and a housing track 38. The housing track 38 may comprise a recessed portion of the housing 10 attached to the housing shoulder 36 at the end opposite the housing opening (not shown). The lid 14 may be mounted on the housing track 38. The lid 14 may slide along the housing track 38 relative to the housing 10, The housing 10 may also comprise a stopping notch (not shown) that prevents the lid from sliding off along the housing track 38 in an axial direction, away from the housing shoulder 36, and off of the housing 10 altogether. Other mounting configurations are also contemplated. In one configuration, the housing track 38 may be recessed from the housing shoulder 36 for a substantially equal to the thickness of the lid. However, it is also contemplated that the housing track may be recessed other distances.

Referring again to Fig, 2, in accordance with one embodiment, not forming part of the invention, the housing 10 may comprise a housing inlet 18 and a dispensing outlet 20. The housing inlet 18 and the dispensing outlet 20 may be located on the top and bottom of the cavity 12 respectively, The housing inlet 18 and the dispensing outlet 20 may be vertically aligned with one another. However, it is also contemplated that the housing inlet 18 and the dispensing outlet 20 may be oriented in other fashions within the cavity 12. The dispensing outlet 20 may be sized to allow at least one test strip to exit the container at a time. The housing inlet 18 may be sized marginally larger than the plunger 30. However, it is also contemplated that the dispensing outlet and housing inlet may be sized in relation to the size of the item to be dispensed and the size of the plunger respectively.

The housing 10 may comprise a cartridge 16 provided in the cavity 12. The cartridge 16 may comprise a molded insert that fits within the cavity 12. The cartridge 16 may also be integrally molded in conjunction with the housing 10. The cartridge 16 may comprise a frame 40 suitable to define a storage chamber 42. The storage chamber 42 may retain the plurality of test strips in a desirable orientation relative to the dispensing outlet and housing inlet.

Referring to Fig. 2, in one embodiment, not forming part of the invention, the storage chamber 42 has a chamber dispensing wall 44 and a chamber biasing wall 46, disposed in a parallel relation to one another on each longitudinal side of the storage chamber 42. The storage chamber 42 may orient the test strips vertically, substantially parallel to the chamber dispensing wall 44, and the chamber biasing wall 46. The frame 40 is operable to support and brace the storage chamber 42. The frame 40 may extend from the floor of the cavity 12 partially up the cavity 12 to provide the storage chamber 42 at a position above the floor of the cavity 12. The frame 40 may provide the storage chamber 42 at a location adjacent to the top of the container 5. It is contemplated that the frame 40 may be sized and oriented to position the storage chamber 42 in a location adjacent the housing inlet 18 and the dispensing outlet 20.

The storage chamber 42 may be shaped to hold a plurality of test strips. The storage chamber 42 may be oriented such that the plurality of test strips are disposed in a stack, their longitudinal side perpendicular to the floor of the cavity 12. The plurality of test strips 24 may be oriented in a vertical or horizontal stack relative to the base of the storage chamber 42. The storage chamber 42 may be sized to accommodate any number of test strips. The storage chamber 42 may be sized to hold any number of test strips ranging from about 10 to about 100 test strips, or from about 30 to about 70 test strips, or about 50 test strips.

The storage chamber 42 may be substantially airtight except for a strip exit 48 and a plunger entrance 50. The strip exit 48 and plunger entrance 50 may be located on the ceiling and floor of the storage chamber 42 respectively. The strip exit 48 and plunger entrance 50 may oriented adjacent to the chamber dispensing wall 44. The strip exit 48 and plunger entrance 50 may both be aligned parallel to the chamber dispensing wall 44. The strip exit 48 plunger entrance 50 may be substantially vertically aligned. It is also contemplated that the strip exit 48 and plunger entrance 50 may be aligned with the interior of the chamber dispensing wall 44.

The strip exit 48 and plunger entrance 50 may be larger in size than a single test strip 24. The strip exit 48 and plunger entrance 50 may vary in size to dispense a variety of test strip sizes, and allow different dispensing mechanisms to be utilized in conjunction with the cartridge 16. The strip exit 48 and plunger entrance 50 may be substantially rectangular in shape.

A biasing member 22 may be disposed within the cartridge 16, near the chamber biasing wall 46. The biasing member 22 may urge the plurality of test strips towards the chamber biasing wall 46. The biasing member 22 may comprise a biasing actuator 74 and a biasing platform 76. The biasing actuator 74 may be attached to the chamber biasing wall 46 of the cartridge 16 with a variety of methods, such as co-molding, adhesion, providing an insert, or other attachment means. The biasing actuator 74 may be similarly attached to the biasing platform 76. The biasing actuator 74 may comprise at least one spring. However, it is also contemplated that the biasing actuator 74 may take other forms sufficient to urge the biasing platform 76 towards the chamber dispensing wall 44. In addition, the biasing member 22 may comprise other structures suitable to bias a plurality of test strips towards the chamber dispensing wall 44.

Referring to Fig. 3, the biasing platform 76 may comprise a flat strip operable to interact with the longitudinal wide portion of the test strip stack. In one configuration, the biasing platform 76 may have a width substantially the same as a test strip. Alternatively, the biasing platform 76 may have a width smaller than a test strip. The biasing platform 76 may be manufactured from a range of materials, including, but not limited to, polypropylene, acrylics, metal, plastics, and other materials.

Referring again to Fig. 2, the biasing member 22 may be configured such that as the plunger 30 moves from an unprimed position (Fig. 1B) to a primed position (Fig. 1A), the biasing member 22 urges a single test strip to align with the plunger entrance 50. As the container 5 is then shifted from a primed position to an unprimed position, the newly provided test strip is urged upwards through the dispensing outlet 20.

The plunger 30 may comprise a rigid shaft disposed vertically through the housing inlet 18. In one configuration, the plunger 30 may have a length, such that when the container 5 is in the unprimed position, the plunger 30 extends to the base of the storage chamber 42, but does not displace the plunger entrance seal 26. In an unprimed position (Fig. 1B), the plunger 30 extends substantially into the storage chamber 42, for a distance sufficient to urge a single test strip 24 partially through the dispensing outlet 20. The plunger 30 may be oriented along the cavity biasing wall 32 to open the plunger entrance seal 26, and urge a single test strip 24 through the strip exit seal 28, and partially through the dispensing outlet 20 when the container 5 is in the primed position (Fig. 1A). The plunger 30 may comprise a material selected from the group including plastic, metal, wood, rubber, and other synthetic and natural materials.

The plunger 30 may have dimensions that are marginally smaller than the dimensions of the housing inlet 18 and the plunger entrance 50. The plunger 30 may comprise a rectangular strip having a thickness and width slightly less than that of the test strips to be dispensed. The plunger 30 may be aligned adjacent to the dispensing wall of the cartridge. The plunger 30 may be dimensioned so that as the plunger 30 enters the storage chamber 42, only a single test strip is contacted by the plunger 30. Other sizes and orientations are also contemplated.

Referring to Fig. 3, in accordance with one embodiment, not forming part of the invention, the cartridge 16 may comprise a plunger entrance seal 26 and a strip exit seal 28. The plunger entrance seal 26 and strip exit seal 28 may be provided in the plunger entrance 50 and strip exit 48 respectively. The plunger entrance seal 26 may comprise a flexible material extending along the plunger entrance 50. The plunger entrance seal 26 may comprise a displaceable flap that extends across the plunger entrance 50. The plunger entrance seal 26 may comprise a spring-actuated sealing device, wherein a flexible flap is urged to close the plunger entrance seal maintain an air and moisture ingress seal. The plunger entrance seal 26 may comprise an elastomeric material, or other material suitable to maintain a hermetic seal across the plunger entrance 50. After being displaced by the plunger, the plunger entrance seal may extend across the entire plunger entrance, and provide a hermetic seal.

The strip exit seal 28 may comprise a flexible material extending along the strip exit 48. The strip exit seal 28 may comprise a displaceable flap that extends across the strip exit 48. The strip exit seal 28 may comprise a spring-actuated sealing device, where the flexible flap is urged to close the strip exit 48 and maintain an air and moisture ingress seal. The strip exit seal 28 may comprise an elastomeric material, or other material suitable to maintain a hermetic seal across the strip exit. The strip exit 48 may be configured to reseal after being displaced by the test strip to be dispensed.

Referring generally to Figs. 1A and 1B, in one embodiment, not forming part of the invention, the test strip container 5 comprises a lid 14 mounted on the housing 10 for relative motion to define a primed position (Fig, 1A) and an unprimed position (Fig. 1B).

In one embodiment, not forming part of the invention, the primed position comprises the lid 14 provided at a maximum distance from the housing shoulder 36. The unprimed position comprises the lid 14 provided at a minimum distance from the housing shoulder 36. The minimum distance may comprise the lid 14 abutting the housing shoulder 36 directly. The maximum distance may comprise the lid 14 extended to contact a stopping notch (not shown) provided on the housing track 38. In one configuration, a user may change the container 5 from a primed position (Fig. 1A) to an unprimed position (Fig. 1B) by sliding the lid 14 along the housing track 38 in the direction of the housing shoulder 36, until the lid 14 contacts the housing shoulder 36. Similarly, a user may change the container from an unprimed position to a primed position by sliding the lid 14 along the housing track 38 in the direction opposite of the housing shoulder 36 until it is stopped by the stopper device (not shown). The stopper device may comprise any means for stopping the lid as understood by one of ordinary skill.

Referring to Fig. 3, as the container shifts from the primed position to the unprimed position, the plunger 30 may enter through the plunger entrance 50, and displace the plunger entrance seal 26 to allow the plunger 30 to enter the cartridge 16. The plunger entrance seal 26 may flex inwardly towards the chamber dispensing wall 44. The plunger entrance seal 26 may contact the plunger 30 and minimize the air ingress into the cartridge 16 while the plunger 30 is disposed within the plunger entrance 50.

In one embodiment, not forming part of the invention, the plunger 30 may be disposed within the cartridge 16 when the container is in an unprimed position. In the primed position, the plunger 30 may be provided outside the cartridge 16, oriented to enter the cartridge 16 and urge a single test strip through the dispensing outlet 20. The plunger 30 has a length sufficient to urge a portion of a single test strip 24 partially through the dispensing outlet 20 when the container 5 is changed from a primed to an unprimed position. The test strip 24 may be urged through dispensing outlet 20 a distance that allows a user to sufficiently grip and remove the test strip 24 from the container 5. The test strip 24 may protrude from the dispensing outlet 20 for a length ranging from about 3 mm to about 30 mm, or from about 5 mm to about 25 mm. It is also contemplated that the test strip may protrude from the dispensing outlet 20 other distances suitable to allow a user to grasp and remove a test strip 24 from the container 5.

As the container shifts from primed position to the unprimed position, the test strip 24 may be ejected from the cartridge 16 by the plunger 30. The test strip 24 may push through the strip exit 48, and displace the strip exit seal 28, and exit through the dispensing outlet 20. The strip exit seal 28 may contact the test strip 24 as it leaves the cartridge 16 to minimize any air and moisture ingress.

Once a test strip is removed from the container, the strip exit seal 28 reseals to reestablish the hermetic seal within the cartridge 16. In the unprimed position, the plunger 30 remains disposed partially within the cartridge 16, with the plunger entrance seal 26 pressing against it, operable to hermetically seal the container.

Referring to Fig. 2, in one embodiment, not forming part of the invention, the container 5 comprises a housing inlet seal 64 and a plug seal 66 provided on the plunger 30. The housing inlet seal 64 may interact with the housing inlet 34 when the container 5 is in the primed position to seal the container 5 from air and moisture ingress. The housing inlet seal 64 may comprise a tapered o-ring type seal provided over the plunger 30. However, the housing inlet seal 64 may also comprise other sealing devices operable to provide a hermetic seal in the housing inlet 18 when the container is an unprimed position.

The plug seal 66 may be provided on the part of plunger 30 that extends furthest away from the housing inlet 34 when the container is in an unprimed position. The plug seal 66 may comprise a tapered plug that sealably interacts with the housing inlet 35 when the container 5 is in an unprimed position. The plug seal 66 may comprise a flexible material suitable to provide a seal when the container is in an unprimed position.

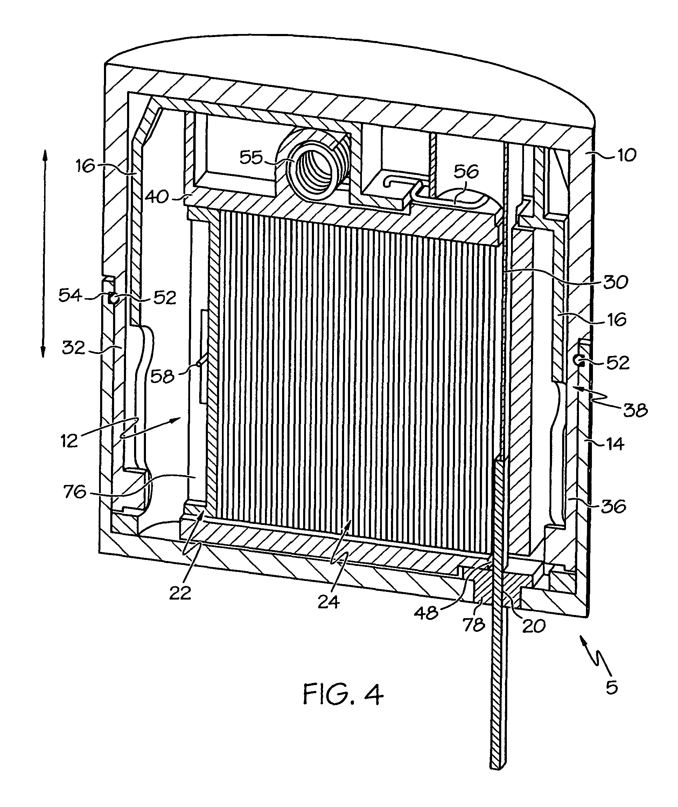

Referring to Fig. 4, in accordance with another embodiment, the container 5 may comprise a housing 10 that defines a cavity 12, a lid 14, and a slidable seal 52. The housing 10 comprises a cartridge 16, a plunger 30, a dispensing outlet 20 and a biasing member 22. In this embodiment, the cartridge has only two locations of potential air and moisture ingress, the junction between the lid 14 and the housing track 38 , and the dispensing outlet 20. The slidable seal 52 may be provided on the inside diameter of the lid 14, and sealably interacts with the housing track 38 to form a hermetic seal within the volume(s) contained by the housing 10 and the portion of the lid beyond the slidable seal 52.

The slidable seal 52 may be located on the inside diameter of the lid 14, only marginally above the open face of the lid 14. The lid comprises a seal gap 54 sized to accommodate a range of slidable seals 52. The seal gap 54 may be sized to accommodate a range of seal sizes, suitable to provide a hermetic seal. The slidable seal 52 should be selected to allow the lid 14 to slide along the housing track 38, and change the container from a primed position to an unprimed position.

Referring to Fig. 4, as the lid 14 slides along the housing track 38 towards the housing shoulder 36 from a primed position to an unprimed position, the plunger 30 may contact a single test strip 24 and urge it partially through the dispensing outlet 20. As the container is transitioned from the unprimed position to the primed position, the plunger 30 moves upward towards the plunger entrance 50 until the distal end of the plunger 30 engages the test strip 24. As the container 5 is transitioned from the primed position to the unprimed position, the test strip is pushed through the strip exit 48 . The test strip may protrude from the dispensing outlet 20 a distance that allows a user to sufficiently grip and remove the test strip 24 from the container 5. The test strip 24 may protrude from the dispensing outlet 20 for a length ranging from about 3 mm to about 30 mm, or from about 5 mm to about 25 mm. It is also contemplated that the test strip may protrude from the dispensing outlet 20 other lengths suitable to allow a user to grasp and remove a test strip from the container.

Referring to Fig. 4, the slidable seal 52 may be provided in a seal gap 54 within the lid 14. Alternatively, the slideable seal may be provided on the outside diameter of the housing track in a seal gap similarly provided. In addition, other slidable seal configurations are also contemplated. The slidable seal 52 may comprise a range of different materials. The slidable seal 52 may comprise a rubber, synthetic rubber, elastomeric materials, and other materials, synthetic and natural operable to form a hermetic seal. The slidable seal 52 may comprise an o-ring type seal.

The slidable seal 52 may provide friction between the housing 10 and the lid 12. The friction may be enough to prevent the lid from sliding along the housing without actuation of the container 5 by a user. In addition, the cartridge 16 may comprise at least two positional notches that define an unprimed position and a primed position. The at least two positional notches may interact with a positional knob provided on the housing to retain the container in a primed and unprimed position until the container is actuated by a user. The positional knob may interact with the positional notches where the positional knob may slide out of the positional notch when a certain force threshold is met.

Referring again to Fig. 4, in accordance with one embodiment, the container 5 may comprise a dispensing outlet seal member 78. The dispensing outlet seal member 78 may cooperate with the dispensing outlet 20 to form a hermetic seal within the container 5. The dispensing outlet seal member 78 may comprise a duckbill seal, that allows a single test strip to pass through the seal. The dispensing outlet seal member 78 may reseal after each test strip 24 is dispensed, in order to maintain the hermetic seal within the container 5. The dispensing outlet seal member 78 may also take other configurations operable to reseal after the test strip is dispensed. The dispensing outlet seal member 78 may also be manufactured from a range of materials known to those skilled in the art including, but not limited to, rubber, plastic, and other synthetic and natural materials suitable to provide a passable outlet forming a substantially moisture-proof and air tight barrier over the dispensing outlet 20.

Referring further to Fig. 4, the container 5 may comprise a spring 55 provided in the cavity 12. The spring 55 may be located in the side of the cavity adjacent the cavity dispensing wall 34. The spring 55 may be statically mounted in the cavity 12. In one configuration, the spring 55 may be provided in the bottom of the cavity 12, integrated with the frame 40. The spring 55 may cooperate with the plunger 30 to longitudinally urge the plunger 30 towards the dispensing outlet 20.

In one embodiment, the spring 55 may comprise a torsion spring having a plunger leg 56 and a biasing leg 58. The biasing leg 58 may extend along the cavity dispensing wall 34, and urge the biasing member 22 towards the chamber biasing wall 46. Simultaneously, the plunger leg 56 may urge the plunger 30 upwards towards the dispensing outlet 20. The spring 55 may comprise any material suitable to provide adequate tension to the container in order to simultaneously urge the biasing member 22 towards the chamber dispensing wall 44, and urge the plunger 30 towards the dispensing outlet 20. In one embodiment, the biasing actuator may be replaced by the biasing leg 58 of the spring 55. The biasing leg 58 may contact the biasing platform 76 and urge the biasing platform towards the cavity biasing wall 32.

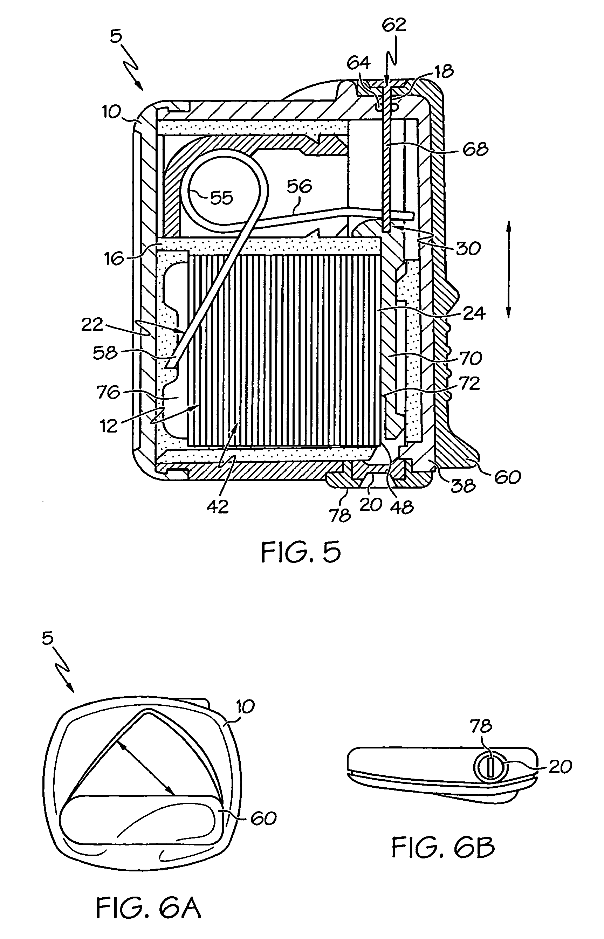

Referring to Fig. 5, in accordance with another embodiment, the container 5 may comprise a housing 10 defining a cavity 12, and a sliding member 60 mounted on the housing 10 to provide for relative motion defining a primed and an unprimed position. In this embodiment, it is contemplated that no lid is included, and the housing 10 is completely encased and hermetically sealed, other than the housing inlet 18 and the dispensing outlet 20.

The sliding member 60 may interact with the plunger 30, such that when the sliding member 60 is moved from a primed position to an unprimed position, the plunger 30 may move longitudinally within the cavity 12 about the same distance as the sliding member 60. The sliding member 60 may connect to the plunger 30 via a plunger mount 62. The plunger mount 62 may connect the sliding member 60 to the plunger 30 in a manner sufficient to urge the plunger 30 towards the dispensing outlet 20 when the sliding member 60 moves from a primed position to an unprimed position. In one configuration, the plunger leg 56 of the spring 55 may be connected to the plunger to ensure that the plunger 30 stays in the unprimed position once a test strip 24 is dispensed.

The plunger 30 may urge a single test strip 24 partially through the dispensing outlet 20 when the sliding member changes from a primed position to an unprimed position. The test strip may protrude from the dispensing outlet a distance that allows a user to sufficiently grip and remove the test strip 24 from the container 5. The test strip 24 may protrude from the dispensing outlet 20 for a length ranging from about 3 mm to about 30 mm, or from about 5 mm to about 25 mm. It is also contemplated that the test strip may protrude from the dispensing outlet 20 other lengths suitable to allow a user to grasp and remove a test strip from the container.

The plunger mount 62 may cooperate with the housing inlet 30 in a manner sufficient to provide a hermetic seal when the container 5 is in an unprimed position. The plunger mount 62 may be complementarily shaped to sit within the housing inlet 30. The plunger mount 62 may include a housing inlet seal 64, that sealably interacts with the housing inlet 18 to provide a hermetic seal. The housing inlet seal 64 may be integrally mounted in conjunction with the plunger mount 62. In one embodiment, the plunger mount 62 may be integrated with the plunger 30, so they are provided as a unitary component. Alternatively, the housing inlet seal 64 may be radially disposed on the plunger mount 62.

The sliding member may comprise a range of materials. The sliding member 60 may be sized sufficient to allow the sliding member 60 to slide over the housing track 38 in a longitudinal fashion. In one configuration, the sliding member 60 has a length that is substantially the same as the length of the housing 10. In another potential configuration, the sliding member 60 may have a length slightly less than the length of the housing 10. In one configuration, the housing track 38 may have a stop that prevents the sliding member 60 from moving past a certain stopping position relative to the housing 10.

Referring to Figs. 6A and 6B, in another embodiment, not forming part of the invention, the sliding member 60 may be pivotally mounted to rotate along the outside of the housing 10, in a manner sufficient to change the container from a primed position to an unprimed position. The arrow in Fig. 6A shows the rotation the sliding member 60 from a primed position to an unprimed position. As the container transitions from a primed position to an unprimed position, the plunger is urged upwards into the storage chamber, sufficient to urge a single test strip 24 through the dispensing outlet 20. The plunger 30 may be a flat flexible wire made of steel or plastic (not shown). As the sliding member 60 is rotated from a primed position to an unprimed position, the plunger engages the end of the test strip, and urges it through a strip exit

As the sliding member 60 is transitioned from a primed to an unprimed position, the sliding member 60 creates translational motion that is transferred through a series of gears to move the plunger 30 from a primed position to an unprimed position (not shown). The sliding member 60 may comprise a series of notches that interacts with the series of gears to provide translational motion to the plunger 30 (not shown). The plunger 30 may also comprise a series of notches that are configured to interact with the series of gears to provide for translational motion as the sliding member is transitioned from a primed to an unprimed position, and vice versa (not shown).

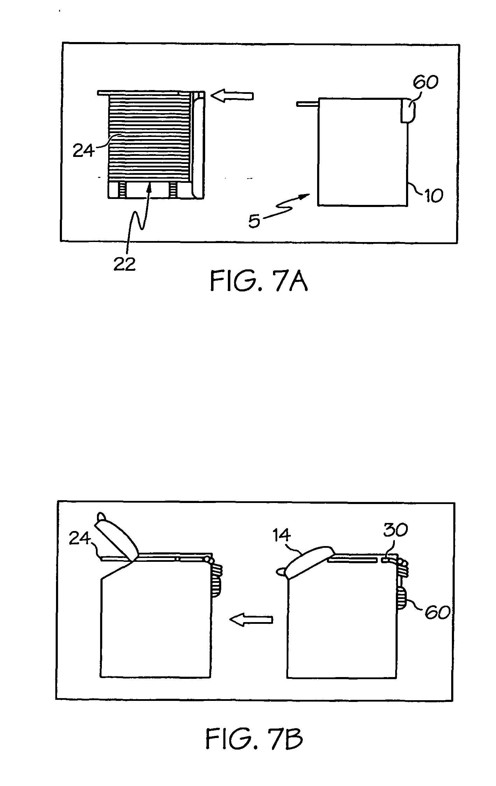

Referring to Figs. 7A and 7B, in another embodiment, not forming part of the invention, the sliding member 60 may provided perpendicularly to the plunger 30. As the sliding member 60 is urged upwards, the plunger 30 is urged axially towards the dispensing outlet as the container is changed from a primed position to an unprimed position. The sliding member 60 may be spring loaded to bias the sliding member 60 in a primed position, In one possible configuration, the sliding member may be sheathed with a thermoelastomer overmold to the housing 10, sufficient to achieve a hermetic seal as the sliding member 60 is rotated from a primed position to an unprimed position.

The plunger 30 may be connected to the sliding member 60 via a flexible wire. As the sliding member 60 changes from a primed position to an unprimed position (Fig. 7B) the plunger is urged towards the dispensing outlet in a horizontal direction contacting a single test strip, and urging it through the dispensing outlet. The plunger 30 may comprise a flexible flat member, comprising metal or plastic. As the sliding member is rotated from a primed position to an unprimed position, the plunger 30 engages the strip and urges the strip forward. The biasing member 22 may be configured to urge a horizontally oriented plurality of test strips 24 upwards towards the plunger 30.

Referring again to Fig. 5, in one embodiment, the plunger 30 comprises an actuation shaft 68 and a strip carrier 70. The strip carrier 70 may be shaped to engage a single test strip and urge it partially through the dispensing outlet 20. The actuation shaft 68 may comprise the portion of the plunger that is disposed outside of the housing when the container 5 is in an unprimed position. The strip carrier 70 may comprise a lifting notch 72 shaped to engage a single test strip. The lifting notch 72 of the strip carrier 70 remains below the bottom of the cartridge 16 when the container 5 is in a primed position. The lifting notch 72 may be disposed above the bottom of the cartridge 16 by a distance sufficient to urge a single test strip 24 partially through the dispensing outlet 20 when the container 5 moves from a primed position to an unprimed position. The actuation shaft 68 and strip carrier 70 may be integrated as a single component, or may be provided as separate components and attached to one another in a manner known to those of ordinary skill.

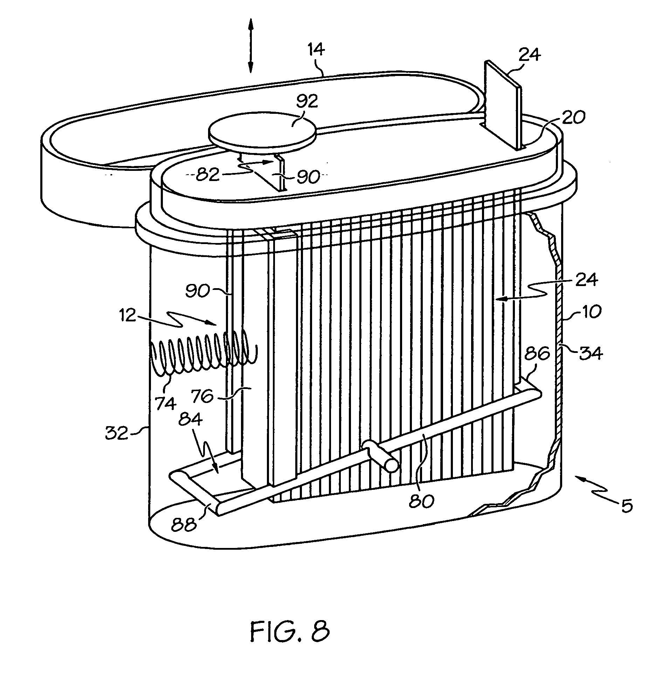

Referring to Fig. 8, in yet another embodiment, not forming part of the invention, the test strip container 5 comprises a housing 10 defining a cavity 12. The housing 10 comprises a dispensing outlet 20, a biasing platform 76 , a lever 80, and a lever actuator 82 located in the cavity 12.

The lever 80 may have a substantially rectangular shape. The lever 80 may have a hole substantially centered in the lever 80 sufficient to define a slot 84 having a substantially rectangular shape. The slot 84 may hold a plurality of test strips in a vertical orientation. The length of the lever 80 may be smaller than the inside dimension of the cavity 12, such that the lever 80 may freely rotate from an primed to an unprimed position.

The lever 80 may be mounted onto the sides of the cavity 12, sufficient to allow the lever 80 to rotate from a primed position to an unprimed position. The lever 80 may be mounted on the walls of the cavity 12 such that the rotation from a primed position to an unprimed position allows the lever 80 to urge a single test strip 24 from the slot 84 at least partially through a dispensing outlet 20. The test strip may protrude from the dispensing outlet 20 a distance that allows a user to sufficiently grip and remove the test strip 24 from the container 5. The test strip 24 may protrude from the dispensing outlet 20 for a length ranging from about 3 mm to about 30 mm, or from about 5 mm to about 25 mm. It is also contemplated that the test strip may protrude from the dispensing outlet 20 other lengths suitable to allow a user to grasp and remove a test strip from the container.

The lever 80 may have a lifting side 86 and a actuation side 88. The lifting side 86 may be oriented to lift a single test strip from the plurality of test strips 24 through the dispensing outlet 20 when the container 5 is in the primed position. The lifting side 86 may be positioned so that it is adjacent to the cavity biasing wall 32, whereas the actuation side 88 may be positioned adjacent to the cavity dispensing wall 34. The arrow (no arrow) indicates the movement of the device, as the container transitions from a primed to an unprimed position.

The container 5 may also comprise a lever actuator 82 functionally connected to the actuation side 88 of the lever 80. The lever actuator 82 may comprise an actuator shaft 90 and an actuator button 92. The actuator button 92 may be provided outside of the cavity 12, on top of the housing 10. The actuator shaft 90 may be primarily disposed within the cavity, and extending outside the cavity 12 to connect to the actuator button 92. The lever actuator 82 urges the actuation side 88 of the lever 80 away from the dispensing outlet 20 and towards the base of the cavity 12. The actuation side 88 may comprise two struts that extend from the actuator button 92 to contact the actuation side 88 in order to rotate the lever 80 from a primed position to an unprimed position. However, it is also contemplated that the lever may be activated in other fashions understood by one of ordinary skill.

The container 5 may also comprise an actuator spring (not shown). The actuator spring may bias the lever actuator 82 away from the lever 80, and allowing the lever 80 return to the primed position. In one configuration, the actuator spring may be provided adjacent to the actuator button 92 to bias the actuator button upwards, and away from the housing 10.

Referring again to Fig. 8, in one embodiment, not forming part of the invention, the biasing platform 76 may be provided within the slot 84 within the lever 80, operable to urge a plurality of test strips towards the lifting side 86 of the lever 80. The biasing platform 76 may urge the plurality of test strips 24 such that when the lever 80 is rotated from an unprimed position, to a primed position, a single strip 24 is urged onto the lifting side of the lever. Subsequently, when the lever 80 is rotated from a primed position to an unprimed position, the test strip 24 is urged partially through the dispensing outlet 20, by a distance sufficient to allow a user to grasp and remove a test strip.

For the purposes of describing and defining the present invention, it is noted that reference herein to a variable being a "function" of a parameter or another variable is not intended to denote that the variable is exclusively a function of the listed parameter or variable. Rather, reference herein to a variable that is a "function" of a listed parameter is intended to be open ended such that the variable may be a function of a single parameter or a plurality of parameters.

It is also noted that recitations herein of "at least one" component, element, etc., should not be used to create an inference that the alternative use of the articles "a" or "an" should be limited to a single component, element, etc.

It is noted that recitations herein of a component of the present disclosure being "programmed" in a particular way, "configured" or "programmed" to embody a particular property, or function in a particular manner, are structural recitations, as opposed to recitations of intended use. More specifically, the references herein to the manner in which a component is "programmed" or "configured" denotes an existing physical condition of the component and, as such, is to be taken as a definite recitation of the structural characteristics of the component.

It is noted that terms like "preferably," "commonly," and "typically," when utilized herein, are not utilized to limit the scope of the claimed invention or to imply that certain features are critical, essential, or even important to the structure or function of the claimed invention. Rather, these terms are merely intended to identify particular aspects of an embodiment of the present disclosure or to emphasize alternative or additional features that may or may not be utilized in a particular embodiment of the present disclosure.

For the purposes of describing and defining the present invention it is noted that the terms "substantially" and "approximately" are utilized herein to represent the inherent degree of uncertainty that may be attributed to any quantitative comparison, value, measurement, or other representation. The terms "substantially" and "approximately" are also utilized herein to represent the degree by which a quantitative representation may vary from a stated reference without resulting in a change in the basic function of the subject matter at issue.

Having described the subject matter of the present disclosure in detail and by reference to specific embodiments thereof, it is noted that the various details disclosed herein should not be taken to imply that these details relate to elements that are essential components of the various embodiments described herein, even in cases where a particular element is illustrated in each of the drawings that accompany the present description. Rather, the claims appended hereto should be taken as the sole representation of the breadth of the present disclosure and the corresponding scope of the various inventions described herein. Further, it will be apparent that modifications and variations are possible without departing from the scope of the invention defined in the appended claims. More specifically, although some aspects of the present disclosure are identified herein as preferred or particularly advantageous, it is contemplated that the present disclosure is not necessarily limited to these aspects.

It is noted that one or more of the following claims utilize the term "wherein" as a transitional phrase. For the purposes of defining the present invention, it is noted that this term is introduced in the claims as an open-ended transitional phrase that is used to introduce a recitation of a series of characteristics of the structure and should be interpreted in like manner as the more commonly used open-ended preamble term "comprising."

## Claims

1. A test strip container (5) for a plurality of test strips (24) comprising:
a housing (10) defining a cavity (12) which holds the plurality of test strips (24);
a dispensing outlet (20);
a dispensing wall (34);
a priming means (14, 60, 82) mounted movably on the housing (10) to define a primed position and an unprimed position therebetween;
a biasing member (22) which urges the plurality of test strips (24) towards the dispensing wall (34) to be aligned with the dispensing outlet (20); and
urging means (30, 80) oriented to urge a single test strip (24) from the plurality of test strips (24) through the dispensing outlet (20) when the priming means (14, 60, 82) is transitioned from the primed position to the unprimed position wherein the priming means is a lid (14), the urging means is a plunger (30), and said container (5) further comprises a cartridge (16) provided within the cavity (12) which holds the plurality of test strips (24), the cartridge (16) comprising the dispensing wall (34) and the dispensing outlet (20), wherein the plunger (30) is oriented to enter the cartridge (16) and urge the single test strip (24) from the plurality of test strips (24) through the dispensing outlet (20) when the lid (14) is transitioned from the primed position to the unprimed position, wherein the housing (10) further comprises a torsion spring (55),
**characterized in that**,
the torsion spring (55) is positioned to bias the plunger (30) towards the unprimed position, the torsion spring (55) comprising a plunger leg (56) and a biasing leg (58), wherein the plunger leg (56) urges the plunger (30) towards the dispensing outlet (20), and the biasing leg (58) urges the biasing member (22) towards the dispensing wall (34).

2. The test strip container (5) according to claim 1, further comprising a housing inlet seal (64) provided on the plunger (30), wherein the housing inlet seal (64) interacts with the housing inlet (18) when the container (5) is in the unprimed position to form a hermetic seal.

3. The test strip container (5) according to claim 1, further comprising a slidable seal (52) positioned on the lid (14) and providing a hermetic seal to a volume defined by the cavity (12) of the housing (10) and the lid (14).

4. The test strip container (5) according to any of the preceding claims, wherein the priming means (14, 60, 82) is slidably mounted on the housing (10).

5. The test strip container (5) according to any of the preceding claims, further comprising a dispensing outlet seal member (78) which is cooperative with the dispensing outlet (20) to form a hermetic seal.

## Patentansprüche

1. Teststreifenbehälter (5) für mehrere Teststreifen (24), umfassend:
ein Gehäuse (10), das einen Hohlraum (12) definiert, in dem mehrere Teststreifen (24) untergebracht sind,
einen Ausgabeauslass (20),
eine Ausgabewand (34),
ein Vorbereitungsmittel (14, 60, 82), das beweglich am Gehäuse (10) montiert ist, um dazwischen eine vorbereitete Position und eine unvorbereitete Position zu definieren,
ein Vorspannmittel (22), das die mehreren Teststreifen (24) zur Ausgabewand (34) hin drängt, so dass sie mit dem Ausgabeauslass (20) fluchten, und ein Drängmittel (30, 80), das so ausgerichtet ist, dass es einen einzelnen Teststreifen (24) aus den mehreren Teststreifen (24) durch den Ausgabeauslass (20) drängt, wenn das Vorbereitungsmittel (14, 60, 82) aus der vorbereiteten Position in die unvorbereitete Position übergeht, wobei das Vorbereitungsmittel ein Deckel (14) ist, das Drängmittel ein Stößel (30) ist und der Behälter (5) ferner ein im Hohlraum (12) vorgesehenes Magazin (16) umfasst, in dem mehrere Teststreifen (24) enthalten sind, wobei das Magazin (16) die Ausgabewand (34) und den Ausgabeauslass (20) umfasst, wobei der Stößel (30) so ausgerichtet ist, dass er in das Magazin (16) eintritt und den einzelnen Teststreifen (24) aus den mehreren Teststreifen (24) durch den Ausgabeauslass (20) drängt, wenn der Deckel (14) aus der vorbereiteten Position in die unvorbereitete Position übergeht, wobei das Gehäuse (10) ferner eine Torsionsfeder (55) umfasst,
**dadurch gekennzeichnet, dass**
die Torsionsfeder (55) so positioniert ist, dass sie den Stößel (30) zur unvorbereiteten Position hin vorspannt, wobei die Torsionsfeder (55) einen Stößelschenkel (56) und einen Vorspannschenkel (58) umfasst, wobei der Stößelschenkel (56) den Stößel (30) zum Ausgabeauslass (20) hin drängt und der Vorspannschenkel (58) das Vorspannelement (22) zur Ausgabewand (34) hin drängt.

2. Teststreifenbehälter (5) nach Anspruch 1, ferner umfassend eine am Stößel (30) vorgesehene Gehäuseeinlassdichtung (64), wobei die Gehäuseeinlassdichtung (64) zur Bildung einer hermetischen Abdichtung mit dem Gehäuseeinlass (18) zusammenwirkt, wenn der Behälter (5) in der unvorbereiteten Position ist.

3. Teststreifenbehälter (5) nach Anspruch 1, ferner umfassend eine verschiebbare Dichtung (52), die am Deckel (14) positioniert ist und eine hermetische Abdichtung für ein Volumen bereitstellt, das durch den Hohlraum (12) des Gehäuses (10) und den Deckel (14) definiert ist.

4. Teststreifenbehälter (5) nach einem der vorhergehenden Ansprüche, wobei das Vorbereitungsmittel (14, 60, 82) verschiebbar am Gehäuse (10) montiert ist.

5. Teststreifenbehälter (5) nach einem der vorhergehenden Ansprüche, ferner umfassend ein Ausgabeauslass-Dichtungselement (78), das zur Bildung einer hermetischen Abdichtung mit dem Ausgabeauslass (20) zusammenwirkt.

## Revendications

1. Récipient de bandelettes réactives (5) pour une pluralité de bandelettes réactives (24) comprenant :
- un boîtier (10) définissant une cavité (12) qui maintient la pluralité de bandelette réactives (24) ;
- une sortie de distribution (20) ;
- une paroi de distribution (34) ;
- un moyen d'amorçage (14, 60, 82) monté mobile sur le boîtier (10) pour définir une position amorcée et une position non amorcée entre elles ;
- un élément de polarisation (22) qui contraint la pluralité de bandelettes réactives (24) vers la paroi de distribution (34) pour qu'elles soient dans l'alignement de la sortie de distribution (20) ; et
- des moyens de contrainte (30, 80) orientés de façon à contraindre une unique bandelette réactive (24) depuis la pluralité de bandelettes réactives (24) à travers la sortie de distribution (20) quand le moyen d'amorçage (14, 60, 82) est passé de la position amorcée à la position non amorcée,
dans lequel le moyen d'amorçage est un couvercle (14), le moyen de contrainte est un piston (30), et ledit récipient (5) comprend en outre une cartouche (16) fournie à l'intérieur de la cavité (12) qui maintient la pluralité de bandes réactives (24), la cartouche (16) comprenant la paroi de distribution (34) et la sortie de distribution (20),
dans lequel le piston (30, 80) orienté de manière à faire entrer la cartouche (16) et l'unique bandelette réactive (24) depuis la pluralité de bandelettes réactives (24) à travers la sortie de distribution (20) quand le couvercle (14, 60, 82) est passé de la position amorcée à la position non amorcée,
dans lequel le boîtier (10) comprend en outre un ressort de torsion (55),
**caractérisé par le fait que** le ressort de torsion (55) est positionné pour polariser le piston (30) vers la position non amorcée, le ressort de torsion (55) comprenant une patte de piston (56) et une patte de polarisation (58), dans lequel la patte de piston (56) contraint le piston (30) vers la sortie de distribution (20), et la patte de polarisation (58) contraint l'élément de polarisation (22) vers la paroi de distribution (34).

2. Récipient de bandelettes réactives (5) selon la revendication 1, comprenant en outre un joint d'entrée de boîtier (64) fourni sur le piston (30), dans lequel le joint d'entrée de boîtier (64) interagit avec l'entrée de boîtier (18) quand le récipient (5) est dans la position amorcée pour former un joint hermétique.

3. Récipient de bandelettes réactives (5) selon la revendication 1, comprenant en outre un joint coulissant (52) positionné sur le couvercle (14) et fournissant un joint hermétique à un volume défini par la cavité (12) du boîtier (10) et le couvercle (14).

4. Récipient de bandelettes réactives selon l'une quelconque des revendications précédentes, dans lequel le moyen d'amorçage (14, 60, 82) est monté coulissant sur le boîtier (10).

5. Récipient de bandelettes réactives selon l'une quelconque des revendications précédentes, comprenant en outre un élément de joint de sortie de distribution (78) qui coopère avec la sortie de distribution (20) pour former un joint hermétique.
